# FASCICULE DE BREVET EUROPEEN

(11) **EP 3 387 794 B1**
(45) Date de publication et mention de la délivrance du brevet: **21.10.2020**
(21) Numéro de dépôt: 16825813.5
(22) Date de dépôt: 09.12.2016
(51) Int. Cl.: G06F 21/62, A61B 5/00, H04L 12/28, A61B 5/0476

(54) **PROCEDE ET SYSTEME DE RECUPERATION DE DONNEES DE FONCTIONNEMENT D'UN DISPOSITIF DE MESURE DES ONDES CEREBRALES**
VERFAHREN UND SYSTEM ZUR WIEDERGEWINNUNG VON BETRIEBSDATEN EINER VORRICHTUNG ZUR MESSUNG VON HIRNWELLEN
METHOD AND SYSTEM FOR RECOVERING OPERATING DATA OF A DEVICE FOR MEASURING BRAIN WAVES

(30) Priorité: 09.12.2015 FR 1562080
(43) Date de publication de la demande: 17.10.2018
(73) Titulaire: DREEM, 75009 Paris (FR)
(72) Inventeur: SOULET DE BRUGIERE, Quentin, 33115 Pyla Sur Mer (FR); MERCIER, Hugo, 75008 Paris (FR); TRANZER, Olivier, 75002 Paris (FR); CATANAS, Adrien, 92600 Asnieres-sur-Seine (FR)
(74) Mandataire: Plasseraud IP
(86) Numéro de dépôt international: PCT/FR2016/053307
(87) Numéro de publication internationale: WO 2017/098185

(56) Documents cités:
- WO-A1-2014/138414
- CN-A- 104 434 045
- US-A1- 2013 231 947
- US-A1- 2014 135 612
- PRASHANTH SHYAMKUMAR ET AL: "A Wearable Remote Brain Machine Interface Using Smartphones and the Mobile Network", ADVANCES IN SCIENCE AND TECHNOLOGY, vol. 85, 13 janvier 2013 (2013-01-13), pages 11-16, XP055292932, DOI: 10.4028/www.scientific.net/AST.85.11
- ARKADIUSZ STOPCZYNSKI ET AL: "The Smartphone Brain Scanner: A Portable Real-Time Neuroimaging System", PLOS ONE, vol. 9, no. 2, 5 février 2014 (2014-02-05) , page e86733, XP055293537, DOI: 10.1371/journal.pone.0086733

## Description

### DOMAINE DE L'INVENTION

La présente invention est relative aux procédés et systèmes de récupération de données de fonctionnement d'un dispositif de mesure des ondes cérébrales d'une personne.

### ARRIERE-PLAN DE L'INVENTION

On connait des dispositifs permettant de mesurer les ondes cérébrales d'une personne, notamment au cours d'une période de sommeil, de travail ou de loisir de ladite personne.

De tels dispositifs de mesure comprennent usuellement un casque ou bandeau muni d'électrodes de mesure d'un encéphalogramme, et par exemple d'un électromyogramme. Le dispositif de mesure est porté par la personne au cours d'une certaine période, par exemple une période de sommeil de l'utilisateur pour un dispositif de suivi de sommeil. De tels dispositifs peuvent en outre agir sur le fonctionnement cérébral de l'utilisateur, par exemple au moyen de stimulateurs sensoriels, par exemple de moyens de stimulation sonore.

Le document WO 2015/17563 décrit un exemple d'un tel dispositif de mesure des ondes cérébrales d'une personne. Le document US2013231947 constitue aussi un document pertinent de l'art antérieur.

Pour traiter les données, en particulier les signaux d'électroencéphalogrammes, recueillies par un tel dispositif de mesure, il est usuellement nécessaire de faire appel à un serveur de traitement car la puissance de calcul et la mémoire requise sont importantes. En outre un serveur de traitement permet de centraliser les données recueillies par une pluralité de dispositif de mesure et conserver et traiter les données issues d'une série d'acquisitions. Il est ainsi possible de mettre en œuvre des algorithmes d'apprentissage ou de calculs statistiques par exemple.

Or, même lorsqu'il n'est pas utilisé, le dispositif de mesure des ondes cérébrales est souvent conservé dans la pièce où se déroule usuellement l'acquisition, par exemple posé durant la journée sur une table de chevet de la chambre à coucher pour un dispositif de suivi de sommeil.

On constate par ailleurs que l'accès au réseau internet n'est pas toujours garanti dans les différentes pièces d'une habitation. Ainsi en particulier le routeur sans-fil d'un réseau Wi-Fi se trouve fréquemment dans une pièce de vie de l'habitation qui peut être éloignée de la chambre à coucher. Ceci peut être choisi par l'utilisateur pour des raisons pratiques et économiques, de sorte limiter le nombre de routeurs Wi-Fi, ou encore parce que l'utilisateur souhaite limiter son exposition aux rayonnements électromagnétiques pendant son sommeil.

De ce fait, le dispositif de mesure peut, en pratique, avoir des difficultés importantes à communiquer avec le réseau internet et donc avec le serveur de traitement. La récupération des données de fonctionnement d'un dispositif de mesure des ondes cérébrales d'une personne sur un serveur de traitement de données peut ainsi être délicate et retardée, à moins d'exiger régulièrement de l'utilisateur une opération manuelle de transfert des données, ce qui est bien évidemment contraignant et chronophage pour ce dernier.

La présente invention a notamment pour but d'améliorer cette situation.

### RESUME DE L'INVENTION

La présente invention est définie par les revendications indépendantes annexées. A cet effet, l'invention a pour premier objet un procédé de récupération de données de fonctionnement d'un dispositif de mesure des ondes cérébrales d'une personne sur un serveur de traitement de données, spécialement destiné à être mis en œuvre par un système comprenant un serveur de traitement de données, un dispositif portable de relai et un dispositif de mesure des ondes cérébrales d'une personne,

le procédé comprenant au moins :
a) une étape de travail au cours de laquelle, pendant une période de travail, on acquiert, au moyen du dispositif de mesure, un signal de mesure (S) représentatif d'un signal physiologique de la personne (P), et on stocke ledit signal de mesure dans une mémoire dudit dispositif de mesure,
b1) une première étape de test de connexion, mise en œuvre après ladite période de travail, au cours de laquelle on détermine si une connexion primaire peut être établie entre le dispositif de mesure et le serveur de traitement de données,
c1) si une connexion primaire peut être établie, une étape de transfert primaire de données de fonctionnement du dispositif de mesure au serveur de traitement de données, au moyen de ladite connexion primaire, lesdites données de fonctionnement étant déterminées à partir du signal de mesure,
b2) si une connexion primaire ne peut pas être établie, une deuxième étape de test de connexion, au cours de laquelle on détermine si une connexion secondaire peut être établie entre le dispositif de mesure et le dispositif portable de relai,
c2) si une connexion secondaire peut être établie, une étape de transfert secondaire de données de fonctionnement du dispositif de mesure au dispositif portable de relai, au moyen de ladite connexion secondaire, lesdites données de fonctionnement étant déterminées à partir du signal de mesure
b3) si une étape de transfert secondaire a été mise en œuvre, une troisième étape de test de connexion, au cours de laquelle on détermine si une connexion tertiaire peut être établie entre le dispositif portable de relai et le serveur de traitement de données,
c3) si une connexion tertiaire peut être établie, une étape de transfert tertiaire des données de fonctionnement du dispositif portable de relai au serveur de traitement de données, au moyen de ladite connexion tertiaire.

Dans des modes de réalisation préférés de l'invention, on peut éventuellement avoir recours en outre à l'une et/ou à l'autre des dispositions suivantes :
- le dispositif portable de relai est un dispositif transportable par un utilisateur, notamment une base, un téléphone portable, un smartphone, une tablette électronique ou un ordinateur portable ;
- la connexion primaire, la connexion secondaire et la connexion tertiaire comportent chacune une communication sans fil ;
- la connexion primaire est mise en œuvre au moyen d'un réseau sans-fil local relié à un réseau étendu, notamment un réseau sans-fil d'entreprise ou un réseau sans-fil domestique relié au réseau internet ;
- la connexion secondaire est une connexion sans fil entre le dispositif de mesure des ondes cérébrales et le dispositif portable de relai, notamment une connexion par ultrasons ou une connexion par radiofréquences telle qu'une connexion Bluetooth ou une communication en champ proche ;
- la connexion tertiaire est mise en œuvre au moins en partie au moyen d'un réseau sans fil tel qu'un réseau cellulaire ou un réseau sans-fil local relié au réseau internet, notamment un réseau sans-fil d'entreprise relié au réseau internet ou un réseau sans-fil domestique relié au réseau internet ;
- le dispositif portable de relai est déplacé entre l'étape de transfert secondaire et l'étape de transfert tertiaire ;
- la deuxième étape de test de connexion comprend une première sous-étape de test au cours de laquelle on détermine si une connexion par radiofréquences peut être établie entre le dispositif de mesure des ondes cérébrales et le dispositif portable de relai,
si une connexion par radiofréquences peut être établie, la connexion secondaire est une connexion par radiofréquences,
si une connexion par radiofréquences ne peut pas être établie, une deuxième sous-étape de test au cours de laquelle on détermine si une connexion par ultrasons peut être établie entre le dispositif de mesure des ondes cérébrales et le dispositif portable de relai,
si une connexion par ultrasons peut être établie, la connexion secondaire est une connexion par ultrasons ;
- les données de fonctionnement transmises du dispositif de mesure des ondes cérébrales au serveur de traitement de données au cours de l'étape de transfert primaire comprennent des données de mesure brutes comprenant le signal de mesure ;
- les données de fonctionnement transmises au cours de l'étape de transfert secondaire et de l'étape de transfert tertiaire comprennent des données de mesure traitées, de préférence ne comprennent pas le signal de mesure (S), encore plus préférentiellement lesdites données de fonctionnement présentent une taille au moins dix fois inférieure à une taille des données de mesure brutes comprenant le signal de mesure (S) ;
- les données de mesure traitées sont déterminées en mettant en œuvre un algorithme de reconnaissance de motifs prédéfinis dans le signal de mesure, notamment de motifs d'ondes lentes, de motifs de fuseaux de sommeil, de motifs associés au réveil et/ou de motifs associés aux mouvements de la personne,
et lesdites données de mesure traitées comprennent des indicateurs relatifs auxdits motifs prédéfinis, notamment un instant de début, une durée, une fréquence et/ou une amplitude d'un motif prédéfinis et/ou un nombre ou une fréquence de motifs prédéfinis au cours de la période de travail ;
- au cours de l'étape de travail, on émet en outre un signal acoustique (A), audible par la personne, et synchronisé avec un motif temporel prédéfini d'onde cérébrale (M1) de la personne,
et les données de fonctionnement transmises au cours de l'étape de transfert primaire comprennent au moins un paramètre de stimulation choisi dans une liste comprenant un instant de début, une durée, une amplitude, un spectre et/ou une référence d'un motif de stimulation acoustique,
de préférence les données de fonctionnement transmises au cours de l'étape de transfert secondaire et au cours de l'étape de transfert tertiaire comprennent également ledit au moins un paramètre de stimulation.

L'invention a également pour objet un système comprenant un serveur de traitement de données, un dispositif portable de relai et un dispositif de mesure des ondes cérébrales d'une personne,
dans lequel le dispositif de mesure comprend
- des moyens d'acquisition aptes, pendant une période de travail, à acquérir au moins un signal de mesure représentatif d'un signal physiologique de la personne (P),
- une mémoire apte à stocker ledit signal de mesure, et
- des moyens de communication aptes à
   - déterminer si une connexion primaire peut être établie entre le dispositif de mesure et le serveur de traitement de données,
   - transférer des données du dispositif de mesure au serveur de traitement de données au moyen d'une connexion primaire,
   - déterminer si une connexion secondaire peut être établie entre le dispositif de mesure et le dispositif portable de relai, et
   - transférer des données du dispositif de mesure au dispositif portable de relai au moyen d'une connexion secondaire,
   dans lequel le dispositif portable de relai comprend des moyens de communication aptes à
   - déterminer si une connexion tertiaire peut être établie entre le dispositif portable de relai et le serveur de traitement de données,
   - transférer des données du dispositif portable de relai au serveur de traitement de données au moyen d'une connexion tertiaire.

L'invention a encore pour objet un dispositif de mesure des ondes cérébrales d'une personne spécialement destiné à être intégré dans un système tel que décrit ci-avant, le dispositif comprenant
- des moyens d'acquisition aptes, pendant une période de travail, à acquérir au moins un signal de mesure représentatif d'un signal physiologique de la personne (P),
- une mémoire apte à stocker ledit signal de mesure, et
- des moyens de communication aptes à
   - déterminer si une connexion primaire peut être établie entre le dispositif de mesure des ondes cérébrales et un serveur de traitement de données d'un système selon la revendication 13,
   - transférer des données du dispositif de mesure des ondes cérébrales audit serveur de traitement de données au moyen d'une connexion primaire,
   - déterminer si une connexion secondaire peut être établie entre le dispositif de mesure des ondes cérébrales et un dispositif portable de relai d'un système selon la revendication 13, et
   - transférer des données du dispositif de mesure des ondes cérébrales audit dispositif portable de relai au moyen d'une connexion secondaire.

Selon une réalisation, le disposition comprend en outre des moyens d'émission conçus pour émettre un signal acoustique, audible par la personne, et synchronisé avec un motif temporel prédéfini d'onde cérébrale de la personne.

Grâce à ces dispositions, entre autre, la récupération des données de fonctionnement du dispositif de mesure des ondes cérébrales d'une personne sur un serveur de traitement est facilitée, est moins contraignante pour l'utilisateur, n'exige de déplacement du dispositif de mesure, ou d'action particulière de l'utilisateur, est plus fiable et n'est pas retardée.

### DESCRIPTION DES FIGURES

D'autres caractéristiques et avantages de l'invention apparaîtront au cours de la description suivante de plusieurs de ses formes de réalisation, données à titre d'exemples non limitatifs, en regard des dessins joints.

Sur les dessins :
- la figure 1 est une vue schématique d'un dispositif de mesure des ondes cérébrales d'une personne selon un mode de réalisation de l'invention,
- la figure 2 est un schéma synoptique d'un système selon un mode de réalisation de l'invention comportant un dispositif de mesure, un dispositif portable de relai et un serveur de traitement de données,
- la figure 3 est un schéma synoptique d'une connexion primaire et d'un transfert primaire de données de fonctionnement entre le dispositif de mesure et le serveur de traitement de données du système de la figure 2, au cours de la mise en œuvre d'un procédé selon un mode de réalisation de l'invention,
- la figure 4 est un schéma synoptique d'une connexion secondaire et d'un transfert secondaire de données de fonctionnement entre le dispositif de mesure et le dispositif portable de relai du système de la figure 2, au cours de la mise en œuvre d'un procédé selon un mode de réalisation de l'invention,
- la figure 5 est un schéma synoptique d'une connexion tertiaire et d'un transfert tertiaire de données de fonctionnement entre le dispositif portable de relai et le serveur de traitement de données du système de la figure 2, au cours de la mise en œuvre d'un procédé selon un mode de réalisation de l'invention,
- la figure 6 est un organigramme illustrant un mode de réalisation d'un procédé de récupération de données de fonctionnement d'un dispositif de mesure des ondes cérébrales d'une personne sur un serveur de traitement de données selon un mode de réalisation de l'invention,
- la figure 7 illustre une forme temporelle d'onde lente cérébrale, un signal acoustique et des motifs temporels prédéfinis selon un exemple de réalisation de l'invention.

Sur les différentes figures, les mêmes références désignent des éléments identiques ou similaires.

### DESCRIPTION DETAILLEE

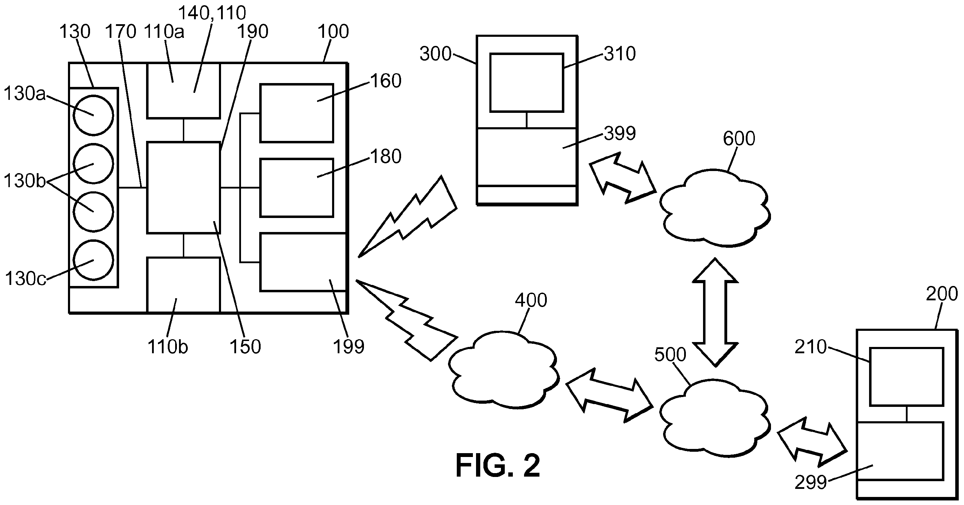

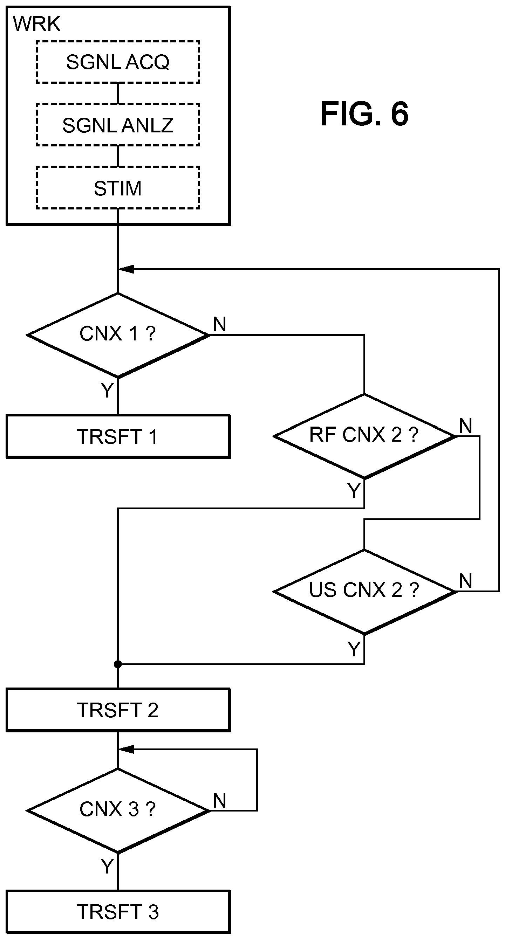

Comme cela est illustré notamment sur les figures 2 et 6, l'invention a pour objet un système 1 comprenant un dispositif de mesure 100 des ondes cérébrales d'une personne, un serveur de traitement de données 200 et un dispositif portable de relai 300.

Le système 1 est apte à mettre en œuvre un procédé de récupération de données de fonctionnement du dispositif de mesure des ondes cérébrales d'une personne P sur le serveur de traitement de données qui est notamment illustré sur la figure 6.

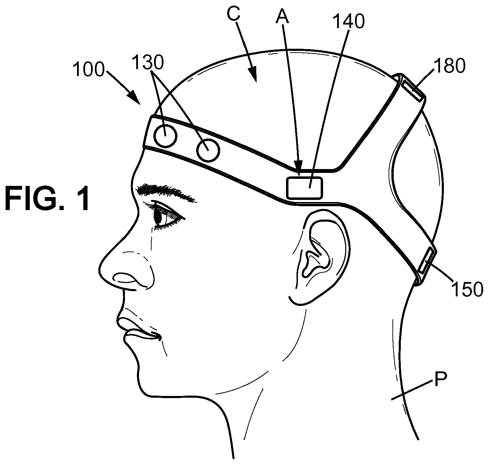

Le dispositif 100 est illustré sur les figures 1 et 2 et est par exemple apte à être porté par la personne P, par exemple sur la tête de la personne P.

A cette fin, le dispositif 100 peut comporter un ou plusieurs éléments supports 120 aptes à entourer au moins partiellement la tête de la personne P de sorte à y être maintenu. Les éléments supports 120 prennent par exemple la forme d'une ou plusieurs branches pouvant être disposées de sorte à entourer la tête de la personne P pour y maintenir le dispositif 100.

Le dispositif 100 peut également être divisé en un ou plusieurs éléments, aptes à être porté sur différentes parties du corps de la personne P, par exemple sur la tête, au poignet ou encore sur le torse.

Le dispositif 100 comporte des moyens d'acquisition 130 d'au moins un signal de mesure et au moins une mémoire 160. Le dispositif 100 peut également comporter des moyens d'analyse 150 du signal de mesure. Le dispositif 100 peut enfin comporter des moyens d'émission 140 conçus pour émettre un signal acoustique audible par la personne P comme cela sera décrit plus loin.

Le dispositif est par exemple adapté pour être porté par la personne P au cours d'une période de travail qui peut s'étendre sur une durée de plusieurs minutes à plusieurs heures, par exemple au moins huit heures.

Par « période de travail », on entend une période au cours de laquelle le dispositif de mesure est actif et met en œuvre une opération de travail prédéfinie, par exemple une acquisition d'un signal de mesure S représentatif d'un signal physiologique de la personne P. La personne P peut, quant à elle, être inactive, par exemple endormie pendant la période de travail. Le dispositif de mesure peut en outre mettre en œuvre d'autres opérations, par exemple d'analyse ou de transmission de données, hors de la période de travail.

La période de travail peut par exemple correspondre à une période de sommeil de la personne P, notamment lorsque le dispositif de mesure est un dispositif de suivi et/ou de stimulation de sommeil.

Le dispositif 100 peut en outre comporter une batterie 180. La batterie 180 peut être notamment apte à alimenter les moyens d'acquisition 130, les moyens d'émission 140 et les moyens d'analyse 150, la mémoire 160 et le module de communication 7.

La batterie 180 est par exemple apte à fournir de l'énergie sans être rechargée sur toute la durée de la période de travail, par exemple sur une durée de plusieurs heures sans devoir être rechargée, par exemple au moins huit heures.

Le dispositif 100 peut en particulier fonctionner de manière autonome pendant la période de travail.

Par « autonome », on entend que le dispositif peut fonctionner pendant la période de travail, et notamment mettre en œuvre des opérations d'acquisition et/ou de stimulation des ondes cérébrales telles que décrites ci-après, sans communiquer avec le serveur de traitement 200, notamment sans communiquer avec le serveur de traitement 200. En particulier on entend que le dispositif peut fonctionner pendant la période de travail sans avoir besoin d'être rechargé en énergie électrique et sans avoir besoin d'être relié structurellement à un dispositif extérieur comme un élément de fixation ou une alimentation électrique.

De cette manière le dispositif 100 est apte à être utilisé dans la vie quotidienne d'une personne P sans imposer de contraintes particulières.

Pour permettre la mise en œuvre des opérations d'acquisition et/ou de stimulation des ondes cérébrales, les moyens d'acquisition 130, les moyens d'émission 140, les moyens d'analyse 150 et la mémoire 160 sont par ailleurs reliés fonctionnellement entre eux et aptes à échanger des informations et des commandes.

A cette fin, les moyens d'acquisition 130, les moyens d'émission 140, les moyens d'analyse 150 et la mémoire 160 sont montés sur l'élément support 120 de sorte à être proches les uns des autres si bien que la communication entre ces éléments 130, 140, 150, 160 est particulièrement rapide et à haut débit. La batterie 180 peut également être montée sur l'élément support 120.

La mémoire 160 peut être montée de manière permanente sur l'élément support 120 ou peut être un module amovible, par exemple une carte mémoire telle qu'une carte SD (acronyme anglo-saxon du terme « Secure Digital »).

La mémoire 160 est apte à enregistrer des données de fonctionnement du dispositif 100 qui seront détaillées dans la suite de la description et peuvent comprendre au moins l'un des éléments suivants : des données de mesure brute comportant un signal de mesure S acquis par les moyens d'acquisition 130, des données de mesure traitées déterminées à partir du signal de mesure S.

La mémoire 160 est apte à être mise à jour dynamiquement au cours du fonctionnement du dispositif 100.

L'étape de travail est illustrée sur la figure 6 et peut ainsi comprendre tout d'abord une sous-étape d'acquisition d'au moins un signal de mesure S au moyen des moyens d'acquisition 130.

Le signal de mesure S peut notamment être représentatif d'un signal électrique physiologique E de la personne P.

Le signal électrique physiologique E peut par exemple comporter un électroencéphalogramme (EEG), un électromyogramme (EMG), un électrooculogramme (EOG), un électrocardiogramme (ECG) ou tout autre biosignal mesurable sur la personne P.

A cette fin, les moyens d'acquisition 130 comportent par exemple une pluralité d'électrodes 130 aptes à être en contact avec la personne P, et notamment avec la peau de la personne P pour acquérir au moins un signal de mesure S représentatif d'un signal électrique physiologique E de la personne P.

Le signal électrique physiologique E comporte avantageusement un électroencéphalogramme (EEG) de la personne P.

A cette fin, dans un mode de réalisation de l'invention, le dispositif 100 comporte au moins deux électrodes 130 dont au moins une électrode de référence 130a et au moins une électrode de mesure EEG 130b.

Le dispositif 100 peut comporter en outre une électrode de masse 130c.

Dans un mode de réalisation particulier, le dispositif 100 comporte au moins trois électrodes de mesure EEG 130c, de sorte à acquérir des signaux électriques physiologiques E comportant au moins trois canaux de mesure d'électroencéphalogramme.

Les électrodes de mesure EEG 130c sont par exemple disposées à la surface du cuir chevelu de la personne P.

Dans d'autres modes de réalisation, le dispositif 100 peut comporter en outre une électrode de mesure l'EMG et, éventuellement, une électrode de mesure EOG.

Les électrodes de mesure 130 peuvent être des électrodes réutilisables ou des électrodes jetables. Avantageusement, les électrodes de mesure 130 sont des électrodes réutilisables de sorte à simplifier l'utilisation quotidienne du dispositif.

Les électrodes de mesure 130 peuvent être, notamment, des électrodes sèches ou des électrodes recouvertes d'un gel de contact. Les électrodes 130 peuvent également être des électrodes textiles ou silicone.

Les moyens d'acquisition 130 peuvent également comporter des dispositifs d'acquisition de signaux de mesure S non uniquement électrique.

Un signal de mesure S peut ainsi être, de manière générale, représentatif d'un signal physiologique de la personne P.

Le signal de mesure S peut en particulier être représentatif d'un signal physiologique de la personne P non électrique ou non-totalement électrique, par exemple un signal de travail cardiaque, tel qu'un rythme cardiaque, une température corporelle de la personne P ou encore des mouvements de la personne P.

A cette fin, les moyens d'acquisition 130 peuvent comporter un détecteur de rythme cardiaque, un thermomètre corporelle, un accéléromètre, un capteur de respiration, un capteur de bio-impédance ou encore un microphone.

Les moyens d'acquisition 130 peuvent encore comporter des dispositifs d'acquisition de signaux de mesure S représentatifs de l'environnement de la personne P.

Le signal de mesure S peut ainsi être représentatif d'une qualité de l'air environnant la personne P, par exemple un taux de dioxyde de carbone ou d'oxygène, ou encore d'une température ou d'un niveau de bruit ambiant.

Enfin, les moyens d'acquisition 130 peuvent comporter des dispositifs d'entrée utilisateur permettant à la personne P d'entrer une information. Par exemple l'utilisateur peut indiquer un indice subjectif de qualité de nuit. Le signal de mesure S peut alors être représentatif d'une information fournie par la personne P.

Le signal de mesure S ainsi obtenu peut ainsi constituer des données de mesure brute au sens de la présente description.

Par ailleurs, dans un mode de réalisation de l'invention, la sous-étape d'acquisition du signal de mesure S comporte par ailleurs un prétraitement du signal de mesure S.

Le prétraitement du signal de mesure S peut comporter, par exemple au moins l'un des prétraitements suivants :
- un filtrage fréquentiel, par exemple un filtrage fréquentiel et/ou par ondelettes du signal de mesure S dans une gamme de fréquences temporelles d'intérêt, par exemple une gamme de fréquences comprise dans une plage allant de 0.3 Hz à 100 Hz,
- un filtrage fréquentiel et/ou par ondelettes de fréquences parasites du signal de mesure S, par exemple apte à filtrer au moins au moins une fréquence parasite du signal de mesure S, par exemple une fréquence parasite appartenant à une plage de fréquences allant de 0.3 Hz à 100 Hz,
- une élimination d'artefacts prédéfinis du signal de mesure S.

Le prétraitement du signal de mesure S peut également comporter des prétraitements tels que :
- une amplification, par exemple une amplification du signal de mesure S par un facteur allant de 10^3 à 10^6, et/ou
- un échantillonnage du signal de mesure S au moyen d'un convertisseur analogique-digital apte, par exemple, à échantillonner le signal de mesure S avec un taux d'échantillonnage de quelques centaines de Hertz, par exemple 256 Hz ou 512 Hz.

Un tel prétraitement du signal de mesure S peut par exemple être mis en œuvre par un module analogique ou un module numérique des moyens d'acquisition 130. Ainsi notamment, les moyens d'acquisition 130 peuvent comporter des électrodes actives aptes à réaliser l'un des prétraitements détaillés ci-dessus.

Le signal de mesure S obtenu à la suite du prétraitement peut également constituer des données de mesure brute au sens de la présente description.

L'étape de travail du présent procédé peut également comprendre une sous-étape de traitement du signal de mesure S.

La sous-étape de traitement du signal de mesure S permet notamment de déterminer des données de mesure traitées.

Pour mettre en œuvre cette sous-étape de traitement, le dispositif comporte des moyens d'analyse 150 aptes à analyser le signal de mesure S.

Les moyens d'analyse 150 peuvent par exemple mettre en œuvre un ou plusieurs algorithmes de reconnaissances de motifs prédéfinis sur le signal de mesure S par exemple de motifs d'ondes lentes, de motifs de fuseaux de sommeil (ou « spindles »), de motifs de complexesK, ou encore de motifs associés au réveil et/ou de motifs associés aux mouvements de la personne.

Les données de mesure traitées peuvent ainsi comprendre des indicateurs relatifs auxdits motifs prédéfinis, par exemple un instant de début, une durée, une fréquence et/ou une amplitude d'un motif prédéfinis et/ou un nombre ou une fréquence de motifs prédéfinis au cours de la période de travail.

Les données de mesure traitées peuvent également comporter d'autres données synthétiques déterminées à partir du signal de mesure S, par exemple des valeurs moyennes du signal, des moyennes spectrales ou autres indicateurs numériques pouvant être déterminés à partir du signal de mesure S.

Les données de mesure traitées peuvent également comprendre des indicateurs de plus haut niveau comme les phases de sommeil ou les instants de réveil ou de micro-réveil.

Les données de mesure traitées peuvent encore comporter le signal de mesure compressé avec pertes, par exemple une compression par ondelettes.Par « signal de mesure brut », on entend le signal de mesure S et éventuellement le signal de mesure compressé par un algorithme de compression sans perte, par exemple une compression entropique de type zip.

Les données de mesure traitées sont ainsi déterminées à partir du signal de mesure S et peuvent en particulier ne pas comprendre le signal de mesure S brut lui-même. De cette manière les données de mesure traitées peuvent présenter une taille inférieure à la taille des données de mesure brutes, par exemple une taille au moins dix fois inférieure à la taille des données de mesure brutes ou au moins 100 fois inférieure, en particulier au moins dix fois inférieure à la taille du signal de mesure S.

Dans un premier exemple de réalisation, un spectre fréquentiel du signal de mesure S peut être déterminé. Les formes prédéfinies sont alors déterminées à partir d'une variation d'énergie du spectre fréquentiel dans des bandes de fréquences prédéfinies tel que par exemple une bande de fréquence des ondes alpha (8 12 Hz), beta (>12 Hz), delta (<4Hz) ou encore thêta (4 7 Hz).

Une énergie du spectre fréquentiel dans une ou plusieurs desdites bandes de fréquences peut être calculée, par exemple en utilisant une transformée de fourrier rapide à court terme.

Dans un autre exemple de réalisation, éventuellement combinable avec le premier exemple de réalisation indiquée, les formes prédéfinies peuvent être déterminées de manière directe dans la forme temporelle du signal de mesure S, notamment en recherchant un ou plusieurs motifs prédéfinis dans le signal de mesure S.

Ainsi par exemple des oscillations lentes et des complexeK peuvent être détectés en recherchant des zéros consécutifs espacés de moins d'une seconde environ et en cherchant un maximum pic à pic.

Lorsque ledit maximum pic à pic dépasse un certain seuil, un motif d'onde lente ou de complexeK peut alors être identifié.

Les moyens d'analyse 150 peuvent également analyser un signal de mesure S représentatif d'un niveau de travail musculaire, par exemple un électrooculogramme. Dans ce cas, les moyens d'analyse 150 peuvent par exemple calculer une moyenne glissante d'une variation du mouvement des yeux.

Les moyens d'analyse 150 peuvent également mettre en œuvre un algorithme d'identification automatique à partir du signal de mesure S. Un tel algorithme d'identification automatique est par exemple défini au cours d'une étape d'apprentissage automatique préliminaire.

Par « algorithme d'identification automatique », on entend un algorithme adapté pour identifier et classer automatiquement des motifs dans des données de mesure, en leur associant par exemple une classe, à partir de règles qualitatives ou quantitatives caractérisant les données de mesure.

Ladite classe associée aux données de mesure peut être sélectionnée dans une base de données de classes, ou peut être une valeur interpolée à partir d'une base de données de classes.

Une « classe » peut ainsi être par exemple un identifiant, par exemple un identifiant alphanumérique d'un motif prédéfini, ou encore une valeur numérique, le cas échéant une valeur entière ou réelle.

La classe obtenue peut identifier un motif prédéfini dans le signal de mesure S, par exemple identifier un motif K-complexe ou un fuseau (« spindle »).

Un tel algorithme d'identification automatique peut par exemple mettre en œuvre un réseau de neurones, une machine à vecteurs de support (ou séparateur à vaste marge), un arbre de décisions, une forêt aléatoire d'arbres de décisions, un algorithme génétique ou encore une analyse factorielle, une régression linéaire, une analyse discriminante de Fisher, une régression logistique ou d'autre méthodes connues du domaine de la classification.

Un tel algorithme peut comporter une pluralité de paramètres qui définissent les règles qualitatives ou quantitatives à partir desquelles l'algorithme d'identification automatique peut détecter et classer automatiquement les données de mesure. De tels paramètres sont par exemple les poids de certains neurones ou de tous les neurones pour un algorithme mettant en œuvre un réseau de neurones. Les paramètres de l'algorithme d'identification automatique peuvent par exemple être prédéfinis au cours d'une étape d'apprentissage automatique supervisée, ou plus ou moins déterminée automatiquement, par exemple par la mise au œuvre d'une étape d'apprentissage automatique semi-supervisée, partiellement supervisée, non-supervisée ou par renforcement. La base de données de classes peut également être prédéfinie au cours d'une telle étape d'apprentissage. Une telle étape d'apprentissage automatique peut être mise en œuvre à partir d'un échantillon d'apprentissage de données de mesure.

Enfin, l'étape de travail peut comprendre sous-étape d'émission d'un signal acoustique A constituant une opération de stimulation des ondes cérébrales de la personne P.

A cette fin, le dispositif 100 peut comporter des moyens d'émission 140 conçus pour émettre un signal acoustique A, audible par la personne, et synchronisé avec un motif temporel prédéfini d'onde cérébrale M1 de la personne s'il est estimé que la personne est dans un état d'aptitude à la stimulation.

A cette fin, les moyens d'émission 140 comportent par exemple au moins un transducteur acoustique 110 et une électronique de contrôle 190.

L'électronique de contrôle 190 est notamment apte, en temps réel souple, à recevoir des moyens d'acquisition 130 le signal de mesure S et à commander l'émission par le transducteur acoustique 110 d'un signal acoustique A synchronisé avec un motif temporel prédéfini T d'une onde lente cérébrale de la personne P.

Par « temps réel souple » (en anglais « soft real-time »), on entend une mise en œuvre de l'opération de stimulation telle que des contraintes temporelle sur cette opération, notamment sur la durée ou la fréquence de répétition de cette opération, soient respectées en moyenne sur une durée totale de mise en œuvre prédéfinie, par exemple de quelques heures. On entend notamment que la mise en œuvre de ladite opération puisse à certains moments dépasser lesdites contraintes temporelles tant que le fonctionnement moyen du dispositif 100 et la mise en œuvre moyenne du procédé les respecte sur la durée totale de mise en œuvre prédéfinie. Des limites temporelles peuvent notamment être prédéfinies au-delà desquelles la mise en œuvre de l'opération de stimulation doit être arrêtée ou mise en pause.

Pour permettre une telle mise en œuvre en temps réel souple, une distance maximale entre les moyens d'acquisition 130, les moyens d'émission 140, les moyens d'analyse 150 et la mémoire 160 peut être inférieur à environ un mètre et de préférence inférieure à quelques dizaines de centimètres. De cette manière, une communication suffisamment rapide entre les éléments du dispositif 100 peut être garantie.

Les moyens d'acquisition 130, les moyens d'émission 140, les moyens d'analyse 150 et la mémoire 160 peuvent par exemple être logés dans les cavités de l'élément support 120, clipsés sur l'élément support 120 ou bien encore fixés à l'élément support 120 par exemple par collage, vissage ou tout autre moyen de fixation adapté. Dans un mode de réalisation de l'invention, les moyens d'acquisition 130, les moyens d'émission 140, les moyens d'analyse 150 et la mémoire 160 peuvent être montés sur l'élément support 120 de manière amovible.

Sans un mode de réalisation avantageux de l'invention, l'électronique de contrôle 190 est reliée fonctionnellement aux moyens d'acquisition 130 et au transducteur acoustique 110 par l'intermédiaire de liaisons filaires 170. De cette façon, on diminue l'exposition de la personne P aux rayonnements électromagnétiques.

Le ou les transducteurs acoustiques 110 sont aptes à émettre un signal acoustique A stimulant au moins une oreille interne de la personne P.

Dans un premier mode de réalisation, un transducteur acoustique 110 est un dispositif ostéophonique stimulant l'oreille interne de la personne P par conduction osseuse.

Ce dispositif ostéophonique 110 peut par exemple être apte à être placé à proximité de l'oreille, par exemple au-dessus comme illustré sur la figure 1, notamment sur une zone de peau recouvrant un os crânien.

Dans un second mode de réalisation, le transducteur acoustique 110 est un haut-parleur stimulant l'oreille interne de la personne P par un conduit auditif menant à ladite oreille interne.

Ce haut-parleur peut être disposé à l'extérieur de l'oreille de la personne P ou dans le conduit auditif.

Le signal acoustique A est un signal modulé appartenant au moins partiellement à une gamme de fréquence audibles par une personne P, par exemple la gamme allant de 20Hz à 30 kHz.

L'électronique de contrôle 190 reçoit les signaux de mesure S des moyens d'acquisition 130, éventuellement prétraités comme détaillé ci-avant.

Si les signaux de mesure S reçus par l'électronique de contrôle 190 ne sont pas prétraités, l'électronique de contrôle 190 peut notamment mettre en œuvre l'un et/ou l'autre des prétraitements détaillés ci-avant.

L'électronique de contrôle 190 est ensuite apte à mettre en œuvre une opération de stimulation des ondes cérébrales de la personne P, opération qui va maintenant être décrite plus en détail.

Les ondes cérébrales peuvent notamment être des ondes lentes cérébrales.

Par « onde lente cérébrale », on entend notamment une onde électrique cérébrale de la personne P présentant une fréquence inférieure à 5 Hz et supérieure à 0,3 Hz. Par « onde lente cérébrale », on peut entendre une onde électrique cérébrale de la personne P présentant une amplitude crête à crête comprise par exemple entre 10 et 200 microvolts. Outre les ondes de fréquences très faibles inférieures à 1Hz, on entend ainsi également par onde lente cérébrale, notamment, les ondes delta de fréquences plus élevées (usuellement entre 1,6 et 4 Hz). On peut encore entendre par onde lente cérébrale, tout type d'onde présentant les caractéristiques de fréquence et d'amplitude mentionnées ci-dessus. Ainsi par exemple, les ondes de sommeil de phase 120 dénommées « K-Complexes » peuvent être considérées comme des ondes lentes cérébrales pour l'invention.

De manière générale, la mise en œuvre de l'invention peut par exemple avoir lieu lors d'une phase de sommeil de la personne P (telles qu'identifiées par exemple dans les standards AASM, acronyme de « American Academy of Sleep Medicine »), par exemple une phase de sommeil profond de la personne P (communément appelé stade 3 ou stade 4) ou au cours d'autre phases de sommeil, par exemple lors du sommeil léger de la personne (appelé usuellement stade 2).

L'invention peut être également être mise en œuvre lors d'une phase d'éveil, d'endormissement ou de réveil de la personne P. Les ondes cérébrales peuvent alors différer des ondes lentes cérébrales.

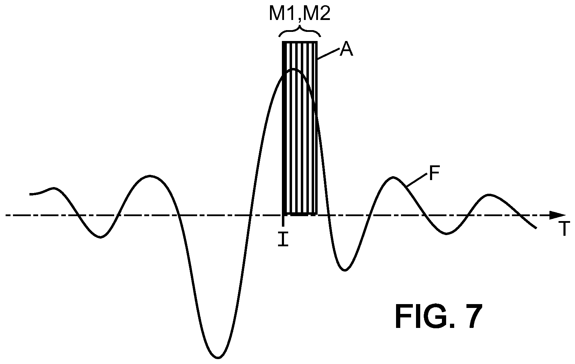

Pour mettre en œuvre l'opération de stimulation des ondes cérébrales, l'électronique de contrôle 190 est par exemple apte, à partir du signal de mesure S, à déterminer tout d'abord une forme temporelle F d'onde lente cérébrale C telle qu'illustrée sur la figure 7.

Dans un premier mode de réalisation, la forme temporelle F est une série de point échantillonnés de valeurs d'amplitudes du signal de mesure S, éventuellement prétraité comme mentionné ci-dessus, ladite série de points de mesure étant éventuellement interpolée ou ré-échantillonnée.

Dans un second mode de réalisation, la forme temporelle F est une série de valeurs d'amplitudes générée par une boucle à phase asservie, ou boucle à verrouillage de phase, (communément désignée par PLL, acronyme du terme anglo-saxon « Phase locked loop »).

La boucle à verrouillage de phase est telle que la phase instantanée de la forme temporelle F en sortie de ladite boucle est asservie sur la phase instantanée du signal de mesure S.

La boucle à verrouillage de phase peut être mise en œuvre par des moyens analogiques ou des moyens numériques.

On comprend donc que la forme temporelle F est une représentation de l'onde cérébrale C qui peut être directement obtenue ou peut être obtenue par une boucle à verrouillage de phase qui permet d'obtenir un signal plus propre. En particulier, la phase instantanée de la forme temporelle F et de l'onde cérébrale C sont synchronisées temporellement. Dans la présente description, on entend donc le cas échéant par « onde cérébrale C » les valeurs prises par la forme temporelle F.

A partir de cette forme temporelle F, l'électronique de contrôle 190 est apte à déterminer au moins un instant temporel I de synchronisation entre un motif temporel prédéfini M1 d'onde lente cérébrale C et un motif temporel prédéfini M2 du signal acoustique A.

Puis, l'électronique de contrôle 190 est apte à commander le transducteur acoustique 110 pour que le motif temporel prédéfini M2 du signal acoustique A soit émis à l'instant temporel I de synchronisation.

Le motif temporel prédéfini M1 d'onde lente cérébrale C est donc un motif de valeurs d'amplitude et/ou de phases de la forme temporelle F qui représente l'onde lente cérébrale C. En particulier, le motif temporel prédéfini M1 peut être une succession de valeurs de phases de la forme temporelle F et peut donc être notamment indépendant de la valeur absolue de l'amplitude de la forme temporelle F.

Le motif temporel prédéfini M1 peut également être une succession de valeurs relatives de l'amplitude de la forme temporelle F. Lesdites valeurs relatives sont par exemple relatives à un maximum d'amplitude de la forme temporelle F prédéfini ou mémorisé.

Dans un mode de réalisation de l'invention, le motif temporel prédéfini M1 peut ainsi par exemple correspondre à un maximum temporel local de l'onde lente cérébrale C, un minimum temporel local de l'onde lente cérébrale C ou encore une succession prédéfinie d'au moins un maximum temporel local et au moins un minimum temporel local de l'onde lente cérébrale C.

Le motif temporel prédéfini M1 peut également correspondre à une portion d'un tel maximum, minimum ou d'une telle une succession, par exemple un front montant, un front descendant ou encore un plateau.

De la même manière, le motif temporel prédéfini M2 du signal acoustique peut être un motif de valeurs d'amplitude et/ou de phases du signal acoustique A.

Dans un premier mode de réalisation, le signal acoustique est par exemple un signal intermittent comme illustré sur la figure 7. Ce signal intermittent est par exemple émis pendant une durée inférieure à une période d'une onde lente cérébrale. La durée du signal intermittent est par exemple inférieure à quelques secondes, préférentiellement inférieure à une seconde.

Dans un exemple donné à titre purement indicatif et non-limitatif, le signal acoustique A est par exemple une impulsion de bruit rose de type 1/f d'une durée temporelle de 50 à 100 millisecondes avec un temps de montée et de descente de quelques millisecondes. Toujours à titre non-limitatif et pour fixer les idées, dans cet exemple le motif temporel prédéfini M1 d'onde lente cérébrale C peut par exemple correspondre à un front montant d'un maximum local de l'onde lente cérébrale C. Le motif temporel prédéfini M2 du signal acoustique A peut alors être par exemple un front montant de l'impulsion de bruit rose. Dans cet exemple, l'instant temporel I de synchronisation entre le motif temporel prédéfini M1 d'onde lente cérébrale C et le motif temporel prédéfini M2 du signal acoustique A peut-être par exemple défini de sorte à ce que le front montant de l'impulsion de bruit rose A et le front montant du maximum local de l'onde lente cérébrale C soit synchronisés, c'est-à-dire concomitants.

Dans un autre mode de réalisation, le signal acoustique A peut être un signal continu. La durée du signal acoustique A peut alors notamment être supérieure à une période de l'onde lente cérébrale C. Par « signal continu », on entend notamment un signal d'une durée grande devant une période de l'onde lente cérébrale C.

Dans ce mode de réalisation, le signal acoustique A peut être modulé temporellement en amplitude, fréquence ou phase et le motif temporel prédéfini M2 du signal acoustique A peut alors être une telle modulation temporelle.

Alternativement, le signal acoustique A continu peut ne pas être modulé temporellement, par exemple d'une manière qui va maintenant être décrite.

Le dispositif 100 peut comporter au moins deux transducteurs acoustiques 110, notamment un premier transducteur acoustique 110a et un second transducteur acoustique 110b comme illustré sur la figure 2. Le premier transducteur acoustique 110a est apte à émettre un signal acoustiques A1 stimulant une oreille interne droite de la personne P. Le second transducteur acoustique 110b est apte à émettre un signal acoustique A2 stimulant une oreille interne gauche de la personne P.

On peut alors en particulier contrôler le premier et le second transducteur acoustique 110a, 110b de telle sorte que les signaux acoustiques A1 et A2 soient des signaux acoustiques binauraux A. A cette fin, les signaux acoustiques A1 et A2 peuvent par exemple être des signaux continus de fréquences différentes.

De tels signaux acoustiques A1, A2 sont connus pour générer des impulsions intermittentes dans le cerveau de la personne P, appelées notamment battements binauraux.

Toujours à titre non-limitatif et pour fixer les idées, dans cet exemple le motif temporel prédéfini M1 d'onde lente cérébrale C peut par exemple, à nouveau, correspondre à un front montant d'un maximum local de l'onde lente cérébrale C. Les motifs temporaux prédéfinis M2 des signaux acoustiques A1, A2 peuvent par ailleurs être des plages des signaux acoustiques A1, A2 correspondant temporellement auxdites impulsions intermittentes générées dans le cerveau de la personne P. Dans cet exemple, l'instant temporel I de synchronisation entre le motif temporel prédéfini M1 d'onde lente cérébrale C et les motifs temporaux prédéfinis M2 des signaux acoustiques A1, A2 peuvent être par exemple définis de sorte à ce qu'une impulsion intermittente générée dans le cerveau de la personne P soit synchronisée temporellement avec le front montant du maximum local de l'onde lente cérébrale C.

La figure 7 illustre un exemple de motifs temporels prédéfini M1 et M2.

L'un et/ou l'autre parmi un niveau sonore, une durée, un spectre et un motif temporel M2 du signal acoustique A peuvent être prédéfinis et enregistrés dans la mémoire 160 du dispositif 100.

Ledit un et/ou autre parmi un niveau sonore, une durée, un spectre et un motif temporel M2 du signal acoustique A peuvent former des données de fonctionnement du dispositif 100.

Plus précisément, les données de fonctionnement peuvent comprendre un ou plusieurs paramètres de stimulation choisis dans une liste comprenant un instant de début, une durée, une amplitude, un spectre et/ou une référence d'un motif de stimulation acoustique du signal acoustique A.

Le signal acoustique A peut ainsi être émis en fonction desdites données de fonctionnement.

Selon les réalisations et selon le motif temporel M1 sélectionné, différents modes de réalisation sont envisageables pour déterminer l'instant temporel I de synchronisation.

De même, l'un et/ou l'autre parmi une phase d'onde cérébrale de la personne et un motif temporel prédéfini d'onde cérébrale M1 de la personne P peuvent être prédéfinis et enregistrés dans la mémoire 160 du dispositif 100.

Ledit un et/ou autre parmi une phase d'onde cérébrale de la personne et un motif temporel prédéfini d'onde cérébrale M1 de la personne P peuvent former des données de fonctionnement du dispositif 100.

Le signal acoustique A peut ainsi être émis de sorte à être synchronisé en fonction desdites données de fonctionnement.

Par ailleurs, pour déterminer l'instant temporel I, l'électronique de contrôle 190 peut par exemple comparer les valeurs d'amplitude du signal de mesure S, éventuellement filtrée et/ou normalisée, avec un seuil d'amplitude.

Dans l'exemple donné ci-avant à titre purement non limitatif, le motif temporel prédéfini M1 d'onde lente cérébrale C correspond à un front montant d'un maximum local de l'onde lente cérébrale C. Un instant temporel I correspond alors à un instant temporel de dépassement du seuil d'amplitude, ou à une durée prédéfinie suivant immédiatement un tel instant de dépassement. L'électronique de contrôle 190 peut ainsi commander le transducteur acoustique 140 pour que le motif temporel prédéfini M2 du signal acoustique A soit synchronisé temporellement avec ledit instant temporel I.

On comprend bien que la rapidité de communication entre les moyens d'acquisition 130, le transducteur acoustique 110 et l'électronique de contrôle 190 permet notamment d'assurer une synchronisation fiable et une mise en œuvre optimale de l'opération de stimulation.

Dans un mode de réalisation dans lequel la forme temporelle F est une série de valeurs d'amplitudes générée par une boucle à phase asservie, il est possible de déterminer ledit instant temporel I à partir de ladite boucle à phase asservie, par détection de seuil ou par prédiction des valeurs futures de la forme temporelle F.

Dans ce mode de réalisation, la forme temporelle F peut notamment être moins bruitée que le signal de mesure S et permettre une détermination facilitée de l'instant temporelle I de synchronisation. De cette manière, il est ainsi plus aisé d'utiliser les valeurs de phases de la forme temporelle F pour identifier l'instant temporelle I.

Comme illustré sur la figure 6, une fois l'étape de travail terminée, le procédé selon l'invention peut alors comporter une première étape de test de connexion.

Cette première étape de test de connexion peut ainsi être mise en œuvre après la période de travail.

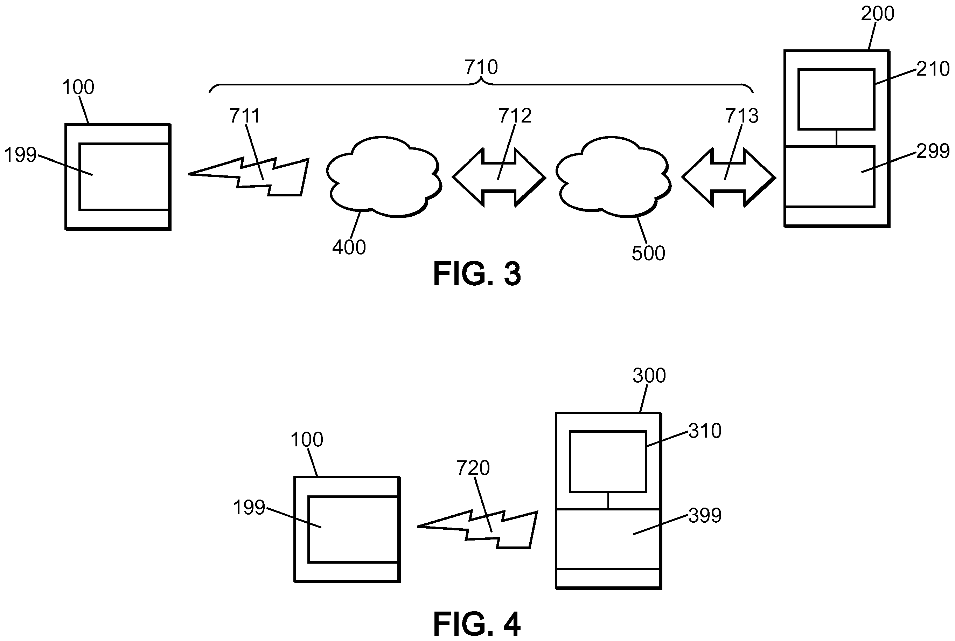

Cette première étape de test de connexion est illustrée sur la figure 3 notamment.

Au cours de la première étape de test de connexion, on détermine si une connexion primaire 710 peut être établie entre le dispositif de mesure 100 et le serveur de traitement de données 200.

A cette fin, le dispositif de mesure 100 peut comporter des moyens de communication 199 et le serveur de traitement de données 200 peut également comporter des moyens de communication 299.

Les moyens de communication 199, 299 du dispositif de mesure 100 et du serveur de traitement de données 200 peuvent être aptes à déterminer si une connexion primaire peut être établie entre le dispositif de mesure 100 et le serveur de traitement de données 200, et à transférer des données du dispositif de mesure 100 au serveur de traitement de données 200, au moyen d'une telle connexion primaire.

Les moyens de communication 199 peuvent être montés sur l'élément support 120 de la manière décrite ci-dessus pour les moyens d'acquisition 130, les moyens d'émission 140 et les moyens d'analyse 150. Les moyens de communication 199 peuvent être commandés par une électronique du dispositif 100, par exemple l'électronique de contrôle 190.

Les moyens de communication 199 comportent en particulier une puce de communication sans-fil.

Les moyens de communication 199 peuvent ainsi comporter un module de communication par radiofréquences, par exemple un module apte à mettre en œuvre une communication en champ proche, une communication Bluetooth et/ou une communication Wi-Fi.

Par Bluetooth, on entend notamment le protocole Bluetooth et le protocole « Bluetooth Low Energy » (BLE).

Les moyens de communication 199 peuvent également comporter un module de communication par ultrasons ou encore un module de communication optique, embarquant par exemple une diode.

Les moyens de communication 299 du serveur de traitement 200 peuvent par exemple être des moyens d'accès au réseau internet, par exemple des moyens de communication filaire comme une carte Ethernet.

La connexion primaire 710 peut être une connexion sans-fil, au moins au départ du dispositif de mesure 100.

Pour cela, la connexion primaire 710 peut être mise en œuvre au moyen d'un réseau sans-fil local 400 relié à un réseau étendu 500.

Le réseau étendu 500 est par exemple le réseau internet.

Le réseau sans-fil local 400 est par exemple un réseau sans-fil d'entreprise ou un réseau sans-fil domestique, en particulier un réseau Wi-Fi relié à internet.

Le dispositif de mesure 100 peut ainsi par exemple chercher à se connecter à un réseau sans-fil domestique et, à partir de ce réseau sans-fil, chercher à se connecter au réseau internet, et par la même au serveur de traitement 200 qui peut également être relié au réseau internet.

La connexion primaire 710 peut ainsi comporter une connexion 711 du dispositif de mesure 100 à un réseau sans-fil local 400, une connexion 712 du réseau sans-fil local 400 à un réseau étendu 500, et une connexion 713 du réseau étendu 500 au serveur de traitement de données 200.

La connexion 711 entre le dispositif de mesure 100 et le réseau sans-fil local 400 peut notamment être une connexion sans fil.

Si une connexion primaire peut être établie, une étape de transfert primaire peut alors être mise en œuvre.

Cette étape de transfert primaire est illustrée sur la figure 3 notamment.

L'étape de transfert primaire peut être mise en œuvre au moyen de ladite connexion primaire.

L'étape de transfert primaire comporte la transmission de données de fonctionnement du dispositif de mesure au serveur de traitement de données.

Les données de fonctionnement peuvent être déterminées à partir du signal de mesure.

Les données de fonctionnement transmises du dispositif de mesure des ondes cérébrales au serveur de traitement de données au cours de l'étape de transfert primaire peuvent notamment comprendre des données de mesure brutes telles que décrites ci-avant, c'est-à-dire comprenant le signal de mesure S.

Si une connexion primaire ne peut pas être établie, le procédé selon l'invention peut alors comporter une deuxième étape de test de connexion illustrée sur la figure 4 notamment.

Au cours de cette deuxième étape de test de connexion, on peut déterminer si une connexion secondaire 720 peut être établie entre le dispositif de mesure 100 et le dispositif portable de relai 300.

Par « connexion secondaire », on entend que cette connexion secondaire est mise en œuvre si la connexion primaire décrite ci-avant n'est pas possible à mettre en œuvre, c'est donc une connexion visant à garantir un fonctionnement résiliant du système.

Le dispositif portable de relai 300 est un dispositif transportable par un utilisateur et apte à communiquer avec le dispositif de mesure et un réseau sans-fil.

Le dispositif portable de relai 300 est par exemple une base, un téléphone portable, un smartphone, une tablette électronique ou un ordinateur portable.

Le dispositif portable de relai 300 peut en particulier comporter des moyens de communication 399.

Les moyens de communication 399 du dispositif portable de relai 300 peuvent comporter une puce de commande ainsi qu'un module de communication sans-fil radiofréquence comportant une antenne, un module de communication par ultrasons comportant un microphone et/ou un module de communication optique comportant par exemple une diode.

Un module de communication sans-fil radiofréquence des moyens de communication 399 peut par exemple être un module apte à mettre en œuvre une communication en champ proche, une communication Bluetooth et/ou une communication Wi-Fi.

Si une connexion secondaire 720 peut être établie, le procédé peut alors comporter une étape de transfert secondaire de données de fonctionnement du dispositif de mesure 100 au dispositif portable de relai 300.

Cette étape de transfert secondaire est illustrée sur la figure 4.

La connexion secondaire 720 est une connexion sans fil entre le dispositif de mesure 100 et le dispositif portable de relai 300. La connexion secondaire 720 peut par exemple être une connexion par ultrasons ou une connexion par radiofréquences, comme une connexion Bluetooth ou une communication en champ proche.

Plus précisément, dans un mode particulier de réalisation de l'invention illustré notamment sur la figure 6, la deuxième étape de test de connexion peut comprendre une première sous-étape de test au cours de laquelle on détermine si une connexion par radiofréquences peut être établie entre le dispositif de mesure 100 et le dispositif portable de relai 300. Une telle connexion radiofréquence peut par exemple être une connexion Bluetooth ou une communication en champ proche.

Si une telle connexion par radiofréquences peut être établie, la connexion secondaire peut alors être une connexion par radiofréquences.

Si une telle connexion par radiofréquences ne peut pas être établie, une deuxième sous-étape de test peut être mise en œuvre au cours de laquelle on détermine si une connexion par ultrasons peut être établie entre le dispositif de mesure 100 et le dispositif portable de relai 300.

Si une connexion par ultrasons peut être établie, la connexion secondaire est alors une connexion par ultrasons.

Dans ce mode de réalisation en particulier, les moyens de communication 399 du dispositif portable de relai 300 peuvent comprendre à la fois un module de communication sans-fil radiofréquence et un module de communication par ultrasons. De manière symétrique, les moyens de communication 199 du dispositif de mesure 100 peuvent comporter à la fois un module de communication sans-fil radiofréquence et un module de communication par ultrasons.

La connexion secondaire peut ainsi être une connexion sans-fil.

L'étape de transfert secondaire peut être mise en œuvre au moyen de ladite connexion secondaire.

L'étape de transfert secondaire comporte la transmission de données de fonctionnement du dispositif de mesure 100 au dispositif portable de relai 300.

Les données de fonctionnement peuvent être déterminées à partir du signal de mesure.

Les données de fonctionnement transmises du dispositif de mesure 100 au dispositif portable de relai 300 au cours de l'étape de transfert secondaire peuvent comprendre des données de mesure traitées telles que décrites ci-avant. En particulier, lesdites données de fonctionnement transmises du dispositif de mesure 100 au dispositif portable de relai 300 peuvent comprendre uniquement des données de mesure traitées et ne pas comprendre le signal de mesure S.

Ainsi par exemple les lesdites données de fonctionnement transmises du dispositif de mesure 100 au dispositif portable de relai 300 peuvent présenter une taille au moins dix fois inférieure à une taille des données de mesure brutes comprenant le signal de mesure S.

De cette manière, il est possible de mettre en œuvre entre le dispositif de mesure 100 et le dispositif portable de relai 300 une communication locale relativement rapide malgré les débits limités des protocoles de communication locaux que sont les connexions Bluetooth, en champ proche ou par ultrasons.

Si une étape de transfert secondaire a été mise en œuvre, le procédé peut alors comporter une troisième étape de test de connexion, au cours de laquelle on détermine si une connexion tertiaire 730 peut être établie entre le dispositif portable de relai 300 et le serveur de traitement de données 200.

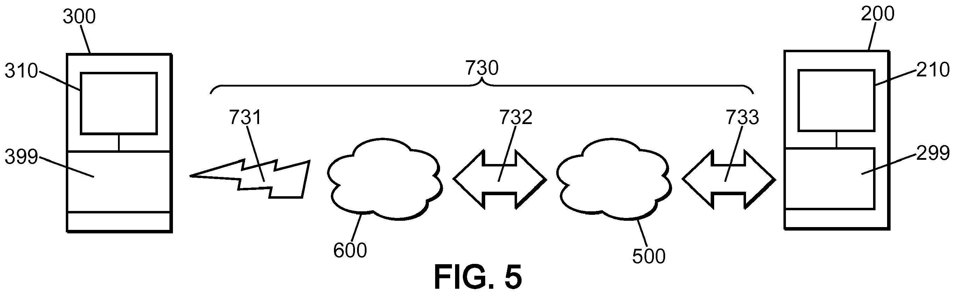

Cette troisième étape de test de connexion est illustrée sur la figure 5.

Par « connexion tertiaire», on entend que cette connexion tertiaire est mise en œuvre si la connexion primaire décrite ci-avant n'est pas possible à mettre en œuvre et si la connexion secondaire a été mise en œuvre, c'est donc une connexion visant à garantir un fonctionnement résiliant du système.

La connexion tertiaire 730 peut être une connexion sans-fil, au moins au départ du dispositif portable de relai 300.

Pour cela, la connexion tertiaire 730 peut être mise en œuvre au moyen d'un réseau sans-fil 600 relié à un réseau étendu 500.

Le réseau sans-fil 600 peut être un réseau cellulaire comme un réseau de téléphonie mobile.

Le réseau sans-fil 600 peut également être un réseau sans-fil local, par exemple un réseau sans-fil d'entreprise ou un réseau sans-fil domestique, en particulier un réseau Wi-Fi relié à internet.

La connexion tertiaire 730 peut ainsi comporter une connexion 731 du dispositif portable de relai à un réseau sans-fil 600, une connexion 732 du réseau sans-fil 600 à un réseau étendu 500, et une connexion 733 du réseau étendu 500 au serveur de traitement de données 200.

La connexion 731 entre le dispositif portable de relai 300 et le réseau sans-fil 600 peut notamment être une connexion sans fil.

Si une connexion tertiaire peut être établie, le procédé peut alors comporter une étape de transfert tertiaire des données de fonctionnement du dispositif portable de relai 300 au serveur de traitement de données 200, au moyen de ladite connexion tertiaire.

La troisième étape de test de connexion et l'étape de transfert tertiaire des données de fonctionnement du dispositif portable de relai au serveur de traitement de données peuvent être mises en œuvre à l'aide des moyens de communication 299, 399 du dispositif portable de relai 300 et du serveur de traitement de données 200.

Les données de fonctionnement transmises du dispositif portable de relai 300 au serveur de traitement de données 200 au cours de l'étape de transfert tertiaire peuvent être identique aux données de fonctionnement transmises du dispositif de mesure 100 au dispositif portable de relai 300 au cours de l'étape de transfert secondaire.

Alternativement, des données additionnelles peuvent être ajoutées par le dispositif portable de relai 300 aux données de fonctionnement transmises du dispositif de mesure 100 au dispositif portable de relai 300 au cours de l'étape de transfert secondaire pour former les données de fonctionnement transmises du dispositif portable de relai 300 au serveur de traitement de données 200 au cours de l'étape de transfert tertiaire.

A cette fin, le dispositif portable de relai peut notamment comporter des moyens de traitement des données 310, notamment au moins une puce de calcul.

Le serveur de traitement 200 peut ainsi obtenir une trace de la période de travail qui s'est écoulée, même s'il ne dispose pas des données de fonctionnement brut.

Comme cela est visible ci-avant, la connexion primaire, la connexion secondaire et la connexion tertiaire peuvent être toutes les trois mises en œuvre, au moins en partie, par des communications sans fil.

Dans un mode de réalisation particulier de l'invention, le dispositif portable de relai 300 peut être déplacé entre l'étape de transfert secondaire et l'étape de transfert tertiaire.

Le dispositif portable de relai 300 peut notamment attendre d'avoir un accès au réseau internet par un canal prédéfini pour mettre en œuvre l'étape de transfert tertiaire.

En particulier, la troisième étape de test de connexion peut consister à déterminer s'il est possible d'établir, entre le dispositif portable de relai et le serveur de traitement de données, une connexion tertiaire qui soit une connexion au travers d'un réseau sans-fil local, par exemple un réseau sans-fil d'entreprise ou un réseau sans-fil domestique, en particulier un réseau Wi-Fi relié à internet.

S'il est uniquement possible d'établir, entre le dispositif portable de relai 300 et le serveur de traitement de données 200, une connexion tertiaire qui soit une connexion à travers un réseau cellulaire comme un réseau de téléphonie mobile, le dispositif portable de relai 300 peut alors choisir d'attendre pour transmettre les données de fonction au serveur de traitement de données 200, de sorte à limiter les coûts pour l'utilisateur.

Dans un mode de réalisation de l'invention dans lequel le dispositif de mesure 100 mets également en œuvre une opération de stimulation des ondes cérébrales, les données de fonctionnement transmises du dispositif de mesure des ondes cérébrales 100 au serveur de traitement de données 200 au cours de l'étape de transfert primaire peuvent en outre comprendre au moins un paramètre de stimulation choisi dans une liste comprenant un instant de début, une durée, une amplitude, un spectre et/ou une référence d'un motif de stimulation acoustique.

Par « une référence d'un motif de stimulation acoustique », on entend par exemple un identifiant alphanumérique d'un motif de stimulation prédéfini.

Dans ce mode de réalisation, les données de fonctionnement transmises du dispositif de mesure 100 au dispositif portable de relai 300 au cours de l'étape de transfert secondaire ainsi que les données de fonctionnement transmises du dispositif portable de relai 300 au serveur de traitement de données 200 au cours de l'étape de transfert tertiaire peuvent également comprendre ledit au moins un paramètre de stimulation.

Dans un mode de réalisation de l'invention, le serveur de traitement de données 200 peut être apte à communiquer avec une pluralité de dispositifs de mesure 100 respectivement aptes à être portés par une pluralité de personnes P.

Le serveur de traitement de données 200 peut ainsi recevoir une pluralité de données de fonctionnement respectivement associées à la pluralité de dispositifs de mesure 100.

Le serveur de traitement de données 200 comporte des moyens de traitement 210, par exemple une ou plusieurs puce(s) de calcul 210, aptes à réaliser un traitement des données de fonctionnement, par exemple aptes à mettre en œuvre des algorithmes d'apprentissage ou de calculs statistiques. Le serveur de traitement de données peut ainsi par exemple déterminer des statistiques ou des indices synthétiques à partir des données de fonctionnement.

## Revendications

1. Procédé de récupération de données de fonctionnement d'un dispositif (100) de mesure des ondes cérébrales d'une personne sur un serveur de traitement de données (200), spécialement destiné à être mis en œuvre par un système (1) comprenant un serveur de traitement de données (200), un dispositif portable de relai (300) et un dispositif (100) de mesure des ondes cérébrales d'une personne,
le procédé comprenant au moins :
a) une étape de travail au cours de laquelle, pendant une période de travail, on acquiert, au moyen du dispositif de mesure (100), un signal de mesure (S) représentatif d'un signal physiologique (E) de la personne (P), et on stocke ledit signal de mesure dans une mémoire (160) dudit dispositif de mesure (100),
b1) une première étape de test de connexion, mise en œuvre après ladite période de travail, au cours de laquelle on détermine si une connexion primaire (710) peut être établie entre le dispositif de mesure (100) et le serveur de traitement de données (200),
c1) si une connexion primaire peut être établie, une étape de transfert primaire de données de fonctionnement du dispositif de mesure (100) au serveur de traitement de données (200), au moyen de ladite connexion primaire (710), lesdites données de fonctionnement étant déterminées à partir du signal de mesure,
b2) si une connexion primaire ne peut pas être établie, une deuxième étape de test de connexion, au cours de laquelle on détermine si une connexion secondaire (720) peut être établie entre le dispositif de mesure (100) et le dispositif portable de relai (300),
c2) si une connexion secondaire peut être établie, une étape de transfert secondaire de données de fonctionnement du dispositif de mesure (100) au dispositif portable de relai (300), au moyen de ladite connexion secondaire (720), lesdites données de fonctionnement étant déterminées à partir du signal de mesure,
b3) si une étape de transfert secondaire a été mise en œuvre, une troisième étape de test de connexion, au cours de laquelle on détermine si une connexion tertiaire (730) peut être établie entre le dispositif portable de relai (300) et le serveur de traitement de données (200),
c3) si une connexion tertiaire peut être établie, une étape de transfert tertiaire des données de fonctionnement du dispositif portable de relai (300) au serveur de traitement de données (200), au moyen de ladite connexion tertiaire (730).

2. Procédé selon la revendication 1, dans lequel le dispositif portable de relai (300) est un dispositif transportable par un utilisateur, notamment une base, un téléphone portable, un smartphone, une tablette électronique ou un ordinateur portable.

3. Procédé selon l'une quelconque des revendications 1 et 2, dans lequel la connexion primaire (710), la connexion secondaire (720) et la connexion tertiaire (730) comportent chacune une communication sans fil.

4. Procédé selon l'une quelconque des revendications 1 à 3, dans lequel la connexion primaire (710) est mise en œuvre au moyen d'un réseau sans-fil local (400) relié à un réseau étendu (500), notamment un réseau sans-fil d'entreprise ou un réseau sans-fil domestique relié au réseau internet.

5. Procédé selon l'une quelconque des revendications 1 à 4, dans lequel la connexion secondaire (720) est une connexion sans fil entre le dispositif (100) de mesure des ondes cérébrales et le dispositif portable de relai (300), notamment une connexion par ultrasons ou une connexion par radiofréquences telle qu'une connexion Bluetooth ou une communication en champ proche.

6. Procédé selon l'une quelconque des revendications 1 à 5, dans lequel la connexion tertiaire (730) est mise en œuvre au moins en partie au moyen d'un réseau sans fil (600) tel qu'un réseau cellulaire ou un réseau sans-fil local relié au réseau internet, notamment un réseau sans-fil d'entreprise relié au réseau internet ou un réseau sans-fil domestique relié au réseau internet.

7. Procédé selon l'une quelconque des revendications 1 à 6, dans lequel le dispositif portable de relai (300) est déplacé entre l'étape de transfert secondaire et l'étape de transfert tertiaire.

8. Procédé selon l'une quelconque des revendications 1 à 7, dans lequel la deuxième étape de test de connexion comprend une première sous-étape de test au cours de laquelle on détermine si une connexion par radiofréquences peut être établie entre le dispositif de mesure (100) des ondes cérébrales et le dispositif portable de relai (300),
si une connexion par radiofréquences peut être établie, la connexion secondaire (720) est une connexion par radiofréquences,
si une connexion par radiofréquences ne peut pas être établie, une deuxième sous-étape de test au cours de laquelle on détermine si une connexion par ultrasons peut être établie entre le dispositif de mesure des ondes cérébrales et le dispositif portable de relai,
si une connexion par ultrasons peut être établie, la connexion secondaire (720) est une connexion par ultrasons.

9. Procédé selon l'une quelconque des revendications 1 à 8, dans lequel les données de fonctionnement transmises du dispositif de mesure (100) des ondes cérébrales au serveur de traitement de données (200) au cours de l'étape de transfert primaire comprennent des données de mesure brutes comprenant le signal de mesure (S) .

10. Procédé selon l'une quelconque des revendications 1 à 9, dans lequel les données de fonctionnement transmises au cours de l'étape de transfert secondaire et de l'étape de transfert tertiaire comprennent des données de mesure traitées, de préférence ne comprennent pas le signal de mesure (S), encore plus préférentiellement dans lequel lesdites données de fonctionnement présentent une taille au moins dix fois inférieure à une taille des données de mesure brutes comprenant le signal de mesure (S).

11. Procédé selon la revendication 10, dans lequel les données de mesure traitées sont déterminées en mettant en œuvre un algorithme de reconnaissance de motifs prédéfinis dans le signal de mesure, notamment de motifs d'ondes lentes, de motifs de fuseaux de sommeil, de motifs associés au réveil et/ou de motifs associés aux mouvements de la personne,
et dans lequel lesdites données de mesure traitées comprennent des indicateurs relatifs auxdits motifs prédéfinis, notamment un instant de début, une durée, une fréquence et/ou une amplitude d'un motif prédéfinis et/ou un nombre ou une fréquence de motifs prédéfinis au cours de la période de travail.

12. Procédé selon l'une quelconque des revendications 1 à 11,
dans lequel au cours de l'étape de travail, on émet en outre un signal acoustique (A), audible par la personne, et synchronisé avec un motif temporel prédéfini d'onde cérébrale (M1) de la personne,
et dans lequel les données de fonctionnement transmises au cours de l'étape de transfert primaire comprennent au moins un paramètre de stimulation choisi dans une liste comprenant un instant de début, une durée, une amplitude, un spectre et/ou une référence d'un motif de stimulation acoustique,
de préférence dans lequel les données de fonctionnement transmises au cours de l'étape de transfert secondaire et au cours de l'étape de transfert tertiaire comprennent également ledit au moins un paramètre de stimulation.

13. Système comprenant un serveur de traitement de données (200), un dispositif portable de relai (300) et un dispositif de mesure (100) des ondes cérébrales d'une personne,
dans lequel le dispositif de mesure (100) comprend
- des moyens d'acquisition (130) aptes, pendant une période de travail, à acquérir au moins un signal de mesure représentatif d'un signal physiologique de la personne (P),
- une mémoire (160) apte à stocker ledit signal de mesure, et
- des moyens de communication (199) aptes à
• déterminer si une connexion primaire (710) peut être établie entre le dispositif de mesure (100) et le serveur de traitement de données (200),
• transférer des données du dispositif de mesure (100) au serveur de traitement de données (200) au moyen d'une connexion primaire (710),
• si une connexion primaire ne peut pas être établie, déterminer si une connexion secondaire (720) peut être établie entre le dispositif de mesure (100) et le dispositif portable de relai (300), et
• transférer des données du dispositif de mesure (100) au dispositif portable de relai (300) au moyen d'une connexion secondaire (720),
dans lequel le dispositif portable de relai (300) comprend des moyens de communication (399) aptes à
• déterminer si une connexion tertiaire (730) peut être établie entre le dispositif portable de relai (300) et le serveur de traitement de données (200),
• transférer des données du dispositif portable de relai (300) au serveur de traitement de données (200) au moyen d'une connexion tertiaire (730).

14. Dispositif de mesure (100) des ondes cérébrales d'une personne spécialement destiné à être intégré dans un système (1) selon la revendication 13, le dispositif comprenant
- des moyens d'acquisition (130) aptes, pendant une période de travail, à acquérir au moins un signal de mesure représentatif d'un signal physiologique de la personne (P),
- une mémoire (160) apte à stocker ledit signal de mesure, et
- des moyens de communication (199) aptes à
• déterminer si une connexion primaire (710) peut être établie entre le dispositif de mesure (100) des ondes cérébrales et un serveur de traitement de données (200) d'un système (1) selon la revendication 13,
• transférer des données du dispositif de mesure (100) des ondes cérébrales audit serveur de traitement de données (200) au moyen d'une connexion primaire (710),
• si une connexion primaire ne peut pas être établie, déterminer si une connexion secondaire (720) peut être établie entre le dispositif de mesure (100) des ondes cérébrales et un dispositif portable de relai (300) d'un système (1) selon la revendication 13, et
• transférer des données du dispositif de mesure (100) des ondes cérébrales audit dispositif portable de relai (300) au moyen d'une connexion secondaire (720). si un transfert secondaire a été mis en œuvre, déterminer si une connexion tertiaire peut être établie entre le dispositif portable de relai et le serveur de traitement de données d'un système (1) selon la revendication 13, et transférer les données du dispositif portable de relai au serveur de traitement de données, au moyen de ladite connexion tertiaire (730).

15. Dispositif selon la revendication 14, comprenant en outre des moyens d'émission (140) conçus pour émettre un signal acoustique (A), audible par la personne, et synchronisé avec un motif temporel prédéfini d'onde cérébrale (M1) de la personne.

## Patentansprüche

1. Verfahren zum Abrufen von Betriebsdaten einer Messvorrichtung (100) für die Gehirnwellen einer Person auf einem Datenverarbeitungsserver (200), das speziell dazu bestimmt ist, von einem System (1) durchgeführt zu werden, das einen Datenverarbeitungsserver (200), eine tragbare Relaisvorrichtung (300) und eine Messvorrichtung (100) für die Gehirnwellen einer Person umfasst,
wobei das Verfahren wenigstens umfasst:
a) einen Arbeitsschritt, bei dem während einer Arbeitsperiode mittels der Messvorrichtung (100) ein Messsignal (S) erfasst wird, das für ein physiologisches Signal (E) der Person (P) repräsentativ ist, und bei dem das Messsignal in einem Speicher (160) der Messvorrichtung (100) gespeichert wird,
b1) einen ersten Verbindungstestschritt, der nach der Arbeitsperiode durchgeführt wird, bei dem bestimmt wird, ob eine primäre Verbindung (710) zwischen der Messvorrichtung (100) und dem Datenverarbeitungsserver (200) hergestellt werden kann,
c1) wenn eine primäre Verbindung hergestellt werden kann, einen primären Übertragungsschritt der Übertragung von Betriebsdaten von der Messvorrichtung (100) zum Datenverarbeitungsserver (200) mittels der primären Verbindung (710), wobei die Betriebsdaten ausgehend von dem Messsignal bestimmt werden,
b2) wenn eine primäre Verbindung nicht hergestellt werden kann, einen zweiten Verbindungstestschritt, bei dem bestimmt wird, ob eine sekundäre Verbindung (720) zwischen der Messvorrichtung (100) und der tragbaren Relaisvorrichtung (300) hergestellt werden kann,
c2) wenn eine sekundäre Verbindung hergestellt werden kann, einen sekundären Übertragungsschritt der Übertragung von Betriebsdaten von der Messvorrichtung (100) zu der tragbaren Relaisvorrichtung (300) mittels der sekundären Verbindung (720), wobei die Betriebsdaten ausgehend von dem Messsignal bestimmt werden,
b3) wenn ein sekundärer Übertragungsschritt durchgeführt wurde, einen dritten Verbindungstestschritt, bei dem bestimmt wird, ob eine tertiäre Verbindung (730) zwischen der tragbaren Relaisvorrichtung (300) und dem Datenverarbeitungsserver (200) hergestellt werden kann,
c3) wenn eine tertiäre Verbindung hergestellt werden kann, einen tertiären Übertragungsschritt der Übertragung der Betriebsdaten von der tragbaren Relaisvorrichtung (300) zum Datenverarbeitungsserver (200) mittels der tertiären Verbindung (730).

2. Verfahren nach Anspruch 1, wobei die tragbare Relaisvorrichtung (300) eine Vorrichtung ist, die von einem Benutzer transportiert werden kann, insbesondere eine Basisstation, ein tragbares Telefon, ein Smartphone, ein elektronisches Tablet oder ein tragbarer Computer.

3. Verfahren nach einem der Ansprüche 1 und 2, wobei die primäre Verbindung (710), die sekundäre Verbindung (720) und die tertiäre Verbindung (730) jeweils eine drahtlose Kommunikation umfassen.

4. Verfahren nach einem der Ansprüche 1 bis 3, wobei die primäre Verbindung (710) mittels eines lokalen drahtlosen Netzwerks (400) implementiert wird, das mit einem Weitverkehrsnetzwerk (500) verbunden ist, insbesondere eines drahtlosen Unternehmensnetzwerks oder eines drahtlosen Heimnetzwerks, das mit dem Internet-Netzwerk verbunden ist.

5. Verfahren nach einem der Ansprüche 1 bis 4, wobei die sekundäre Verbindung (720) eine drahtlose Verbindung zwischen der Messvorrichtung (100) für die Gehirnwellen und der tragbaren Relaisvorrichtung (300) ist, insbesondere eine Ultraschallverbindung oder Hochfrequenzverbindung wie eine Bluetooth-Verbindung oder eine Nahfeldkommunikation.

6. Verfahren nach einem der Ansprüche 1 bis 5, wobei die tertiäre Verbindung (730) zumindest teilweise mittels eines drahtlosen Netzwerks (600) wie eines zellulären Netzwerks oder eines lokalen drahtlosen Netzwerks durchgeführt wird, das mit dem Internet-Netzwerk verbunden ist, insbesondere eines drahtlosen Unternehmensnetzwerks, das mit dem Internet-Netzwerk verbunden ist, oder eines drahtlosen Heimnetzwerks, das mit dem Internet-Netzwerk verbunden ist.

7. Verfahren nach einem der Ansprüche 1 bis 6, wobei die tragbare Relaisvorrichtung (300) zwischen dem sekundären Übertragungsschritt und dem tertiären Übertragungsschritt bewegt wird.

8. Verfahren nach einem der Ansprüche 1 bis 7, wobei der zweite Verbindungstestschritt einen ersten Testunterschritt umfasst, bei dem bestimmt wird, ob eine Hochfrequenzverbindung zwischen der Messvorrichtung (100) für die Gehirnwellen und der tragbaren Relaisvorrichtung (300) hergestellt werden kann,
wobei, wenn eine Hochfrequenzverbindung hergestellt werden kann, die sekundäre Verbindung (720) eine Hochfrequenzverbindung ist,
und wenn keine Hochfrequenzverbindung hergestellt werden kann, einen zweiten Testunterschritt, bei dem bestimmt wird, ob eine Ultraschallverbindung zwischen der Messvorrichtung für die Gehirnwellen und der tragbaren Relaisvorrichtung hergestellt werden kann,
wobei, wenn eine Ultraschallverbindung hergestellt werden kann, die sekundäre Verbindung (720) eine Ultraschallverbindung ist.

9. Verfahren nach einem der Ansprüche 1 bis 8, wobei die Betriebsdaten, die von der Messvorrichtung (100) für die Gehirnwellen zu dem Datenverarbeitungsserver (200) während des primären Übertragungsschritts übertragen werden, Rohmessdaten umfassen, welche das Messsignal (S) umfassen.

10. Verfahren nach einem der Ansprüche 1 bis 9, wobei die während des sekundären Übertragungsschritts und des tertiären Übertragungsschritts übertragenen Betriebsdaten verarbeitete Messdaten enthalten, welche vorzugsweise das Messsignal (S) nicht enthalten, wobei noch bevorzugter die Betriebsdaten eine Größe haben, die mindestens zehnmal kleiner ist als eine Größe der Rohmessdaten, die das Messsignal (S) umfassen.

11. Verfahren nach Anspruch 10, wobei die verarbeiteten Messdaten durch Durchführen eines Algorithmus zum Erkennen vordefinierter Muster in dem Messsignal bestimmt werden, insbesondere langsamer Wellenmuster, Schlafspindelmuster, Muster im Zusammenhang mit dem Erwachen oder/und Muster im Zusammenhang mit den Bewegungen der Person,
und wobei die verarbeiteten Messdaten Indikatoren umfassen, die sich auf die vordefinierten Muster beziehen, insbesondere eine Startzeit, Dauer, Frequenz oder/und Amplitude eines vordefinierten Musters oder/und eine Anzahl oder Häufigkeit vordefinierter Muster während der Arbeitsperiode.

12. Verfahren nach einem der Ansprüche 1 bis 11,
wobei während des Arbeitsschritts auch ein akustisches Signal (A) ausgesendet wird, das von der Person hörbar und mit einem vordefinierten Gehirnwellen-Zeitmuster (M1) der Person synchronisiert ist;
und wobei die während des primären Übertragungsschritts übertragenen Betriebsdaten mindestens einen Stimulationsparameter umfassen, der aus einer Liste ausgewählt ist, die eine Startzeit, eine Dauer, eine Amplitude, ein Spektrum oder/und eine Referenz auf ein akustisches Stimulationsmuster umfasst,
wobei vorzugsweise die während des sekundären Übertragungsschritts und während des tertiären Übertragungsschritts übertragenen Betriebsdaten auch den mindestens einen Stimulationsparameter enthalten.

13. System, umfassend einen Datenverarbeitungsserver (200), eine tragbare Relaisvorrichtung (300) und eine Messvorrichtung (100) für die Gehirnwellen einer Person,
wobei die Messvorrichtung (100) umfasst:
- Erfassungsmittel (130), die dazu geeignet sind, während einer Arbeitsperiode mindestens ein Messsignal zu erfassen, das für ein physiologisches Signal der Person (P) repräsentativ ist;
- einen Speicher (160), der dazu geeignet ist, das Messsignal zu speichern, und
- Kommunikationsmittel (199), die dazu geeignet sind:
• zu bestimmen, ob eine primäre Verbindung (710) zwischen der Messvorrichtung (100) und dem Datenverarbeitungsserver (200) hergestellt werden kann;
• Daten von der Messvorrichtung (100) zum Datenverarbeitungsserver (200) über eine primäre Verbindung (710) zu übertragen,
• falls eine primäre Verbindung nicht hergestellt werden kann, zu bestimmen, ob eine sekundäre Verbindung (720) zwischen der Messvorrichtung (100) und der tragbaren Relaisvorrichtung (300) hergestellt werden kann, und
• Daten von der Messvorrichtung (100) zu der tragbaren Relaisvorrichtung (300) über eine sekundäre Verbindung (720) zu übertragen,
wobei die tragbare Relaisvorrichtung (300) Kommunikationsmittel (399) umfasst, die dazu in der Lage sind:
• zu bestimmen, ob eine tertiäre Verbindung (730) zwischen der tragbaren Relaisvorrichtung (300) und dem Datenverarbeitungsserver (200) hergestellt werden kann;
• Daten von der tragbaren Relaisvorrichtung (300) zum Datenverarbeitungsserver (200) mittels einer tertiären Verbindung (730) zu übertragen.

14. Messvorrichtung (100) für die Gehirnwellen einer Person, die speziell dazu bestimmt ist, in ein System (1) nach Anspruch 13 integriert zu werden, wobei die Vorrichtung umfasst:
- Erfassungsmittel (130), die dazu geeignet sind, während einer Arbeitsperiode mindestens ein Messsignal zu erfassen, das für ein physiologisches Signal der Person (P) repräsentativ ist;
- einen Speicher (160), der dazu geeignet ist, das Messsignal zu speichern, und
- Kommunikationsmittel (199), die dazu geeignet sind:
• zu bestimmen, ob eine primäre Verbindung (710) zwischen der Messvorrichtung für die Gehirnwellen (100) und einem Datenverarbeitungsserver (200) eines Systems (1) gemäß Anspruch 13 hergestellt werden kann,
• Daten von der Messvorrichtung (100) für die Gehirnwellen zu dem Datenverarbeitungsserver (200) mittels einer primären Verbindung (710) zu übertragen,
• wenn eine primäre Verbindung nicht hergestellt werden kann, zu bestimmen, ob eine sekundäre Verbindung (720) zwischen der Messvorrichtung (100) für die Gehirnwellen und einer tragbaren Relaisvorrichtung (300) eines Systems (1) gemäß Anspruch 13 hergestellt werden kann, und
• Daten von der Messvorrichtung (100) für die Gehirnwellen zu der tragbaren Relaisvorrichtung (300) über eine sekundäre Verbindung (720) zu übertragen,
• wenn eine sekundäre Übertragung stattgefunden hat, zu bestimmen, ob eine tertiäre Verbindung zwischen der tragbaren Relaisvorrichtung und dem Datenverarbeitungsserver eines Systems (1) gemäß Anspruch 13 hergestellt werden kann, und die Daten von der tragbaren Relaisverrichtung zu dem Datenverarbeitungsserver mittels der tertiären Verbindung (730) zu übertragen.

15. Vorrichtung nach Anspruch 14, ferner umfassend Sendemittel (140), die dazu ausgelegt sind, ein akustisches Signal (A) auszusenden, das für die Person hörbar ist und mit einem vordefinierten Gehirnwellen-Zeitmuster (M1) der Person synchronisiert ist.

## Claims

1. Method for recovering operating data of a device (100) for measuring a person's brain waves on a data-processing server (200), specially intended to be implemented by a system (1) comprising a data-processing server (200), a portable relay device (300) and a device (100) for measuring a person's brain waves,
the method comprising at least:
a) a working step during which, during a working period, a measurement signal (S) representative of a physiological signal (E) of the person (P) is acquired via the measurement device (100), and said measurement signal is stored in a memory (160) of said measurement device (100),
b1) a first connection-testing step, implemented after said working period, during which it is determined whether a primary connection (710) can be established between the measurement device (100) and the data-processing server (200), c1) if a primary connection can be established, a step of primary transfer of operating data from the measurement device (100) to the data-processing server (200), via said primary connection (710), said operating data being determined on the basis of the measurement signal,
b2) if a primary connection cannot be established, a second connection-testing step, during which it is determined whether a secondary connection (720) can be established between the measurement device (100) and the portable relay device (300),
c2) if a secondary connection can be established, a step of secondary transfer of operating data from the measurement device (100) to the portable relay device (300), via said secondary connection (720), said operating data being determined on the basis of the measurement signal,
b3) if a step of secondary transfer has been implemented, a third connection-testing step, during which it is determined whether a tertiary connection (730) can be established between the portable relay device (300) and the data-processing server (200),
c3) if a tertiary connection can be established, a step of tertiary transfer of the operating data from the portable relay device (300) to the data-processing server (200), via said tertiary connection (730).

2. Method according to claim 1, wherein the portable relay device (300) is a device that can be transported by a user, in particular a base, a mobile telephone, a smartphone, a tablet computer or a laptop computer.

3. Method according to any one of claims 1 and 2, wherein the primary connection (710), the secondary connection (720) and the tertiary connection (730) each include wireless communication.

4. Method according to any one of claims 1 to 3, wherein the primary connection (710) is implemented via a local wireless network (400) connected to a wide area network (500), in particular a corporate wireless network or a home wireless network connected to the internet.

5. Method according to any one of claims 1 to 4, wherein the secondary connection (720) is a wireless connection between the device (100) for measuring the brain waves and the portable relay device (300), in particular a connection via ultrasound or a connection via radio frequencies such as a Bluetooth connection or near-field communication.

6. Method according to any one of claims 1 to 5, wherein the tertiary connection (730) is implemented at least partly via a wireless network (600) such as a cellular network or a local wireless network connected to the internet, in particular a corporate wireless network connected to the internet or a home wireless network connected to the internet.

7. Method according to any one of claims 1 to 6, wherein the portable relay device (300) is moved between the step of secondary transfer and the step of tertiary transfer.

8. Method according to any one of claims 1 to 7, wherein the second connection-testing step comprises a first testing substep during which it is determined whether a connection via radio frequencies can be established between the device (100) for measuring the brain waves and the portable relay device (300),
if a connection via radio frequencies can be established, the secondary connection (720) is a connection via radio frequencies,
if a connection via radio frequencies cannot be established, a second testing substep during which it is determined whether a connection via ultrasound can be established between the device for measuring the brain waves and the portable relay device,
if a connection by ultrasound can be established, the secondary connection (720) is a connection via ultrasound.

9. Method according to any one of claims 1 to 8, wherein the operating data transmitted from the device (100) for measuring the brain waves to the data-processing server (200) during the step of primary transfer comprises raw measurement data comprising the measurement signal (S).

10. Method according to any one of claims 1 to 9, wherein the operating data transmitted during the step of secondary transfer and the step of tertiary transfer comprises processed measurement data, preferably does not comprise the measurement signal (S), even more preferably wherein said operating data has a size at least ten times smaller than a size of the raw measurement data comprising the measurement signal (S).

11. Method according to claim 10, wherein the processed measurement data is determined by implementing an algorithm for recognising predefined patterns in the measurement signal, in particular slow-wave patterns, sleep-spindle patterns, patterns associated with waking up and/or patterns associated with the movements of the person,
and wherein said processed measurement data comprises indicators relative to said predefined patterns, in particular a predefined start time, duration, frequency and/or amplitude of a pattern and/or a number or a frequency of predefined patterns during the working period.

12. Method according to any one of claims 1 to 11,
wherein during the working step, an acoustic signal (A), audible to the person, and synchronised with a predefined brain-wave time pattern (M1) of the person is further emitted,
and wherein the operating data transmitted during the step of primary transfer comprises at least one stimulation parameter chosen from a list comprising a start time, a duration, an amplitude, a spectrum and/or a reference of an acoustic stimulation pattern,
preferably wherein the operating data transmitted during the step of secondary transfer and during the step of tertiary transfer also comprises said at least one stimulation parameter.

13. System comprising a data-processing server (200), a portable relay device (300) and a device (100) for measuring a person's brain waves,
wherein the measurement device (100) comprises
- acquisition means (130) capable, during a working period, of acquiring at least one measurement signal representative of a physiological signal of the person (P),
- a memory (160) capable of storing said measurement signal, and
- communication means (199) capable of
-- determining whether a primary connection (710) can be established between the measurement device (100) and the data-processing server (200),
-- transferring data from the measurement device (100) to the data-processing server (200) via a primary connection (710),
-- if a primary connection cannot be established, determining whether a secondary connection (720) can be established between the measurement device (100) and the portable relay device (300), and
-- transferring data from the measurement device (100) to the portable relay device (300) via a secondary connection (720),
wherein the portable relay device (300) comprises communication means (399) capable of
-- determining whether a tertiary connection (730) can be established between the portable relay device (300) and the data-processing server (200),
-- transferring data from the portable relay device (300) to the data-processing server (200) via a tertiary connection (730).

14. Device (100) for measuring a person's brain waves specially intended to be integrated into a system (1) according to claim 13, the device comprising
- acquisition means (130) capable, during a working period, of acquiring at least one measurement signal representative of a physiological signal of the person (P),
- a memory (160) capable of storing said measurement signal, and
- communication means (199) capable of
-- determining whether a primary connection (710) can be established between the device (100) for measuring the brain waves and a data-processing server (200) of a system (1) according to claim 13,
-- transferring data from the device (100) for measuring the brain waves to said data-processing server (200) via a primary connection (710),
-- if a primary connection cannot be established, determining whether a secondary connection (720) can be established between the device (100) for measuring the brain waves and a portable relay device (300) of a system (1) according to claim 13, and
-- transferring data from the device (100) for measuring the brain waves to said portable relay device (300) via a secondary connection (720)
if a secondary transfer has been implemented, determining whether a tertiary connection can be established between the portable relay device and the data-processing server of a system (1) according to claim 13, and transferring the data from the portable relay device to the data-processing server, via said tertiary connection (730).

15. Device according to claim 14, further comprising emission means (140) designed to emit an acoustic signal (A), audible to the person, and synchronised with a predefined brain-wave time pattern (M1) of the person.
